# EUROPEAN PATENT SPECIFICATION

(11) **EP 3 746 155 B1**
(45) Date of publication and mention of the grant of the patent: **14.10.2026**
(21) Application number: 19746785.5
(22) Date of filing: 31.01.2019
(51) Int. Cl.: A61M 5/178, A61M 5/32, A61J 1/20, A61M 5/46, A61J 1/14, A61M 5/34

(54) **SYRINGE ASSEMBLY**
SPRITZENANORDNUNG
ENSEMBLE SERINGUE

(30) Priority: 02.02.2018 US 201862625560 P
(43) Date of publication of application: 09.12.2020
(73) Proprietor: Becton, Dickinson and Company, Franklin Lakes, NJ 07417-1880 (US)
(72) Inventor: HANG, Tianqi, Franklin Lakes, NJ 07417 (US); SWAJGER, Keith, Franklin Lakes, NJ 07417 (US); BOWEN, Mark, Englewood, FL 34223 (US); JACOBI, Lance, Kearney, NE 68845 (US); DESAI, Niraj, Franklin Lakes, NJ 07417 (US); COLE, Russell, River Vale, NJ 07675 (US); LIMAYE, Amit, Wayne, NJ 07470 (US)
(74) Representative: dompatent
(86) International application number: PCT/US2019/015957
(87) International publication number: WO 2019/152589

(56) References cited:
- EP-A1- 2 857 055
- EP-A2- 1 930 038
- EP-A2- 3 181 167
- WO-A2-2006/099441
- GB-A- 2 461 085
- US-A- 4 994 045
- US-A- 5 002 533
- US-A- 5 611 782
- US-A1- 2004 024 354
- US-A1- 2005 159 706
- US-A1- 2007 208 296
- US-A1- 2016 271 017

## Description

This application claims priority to U.S. Provisional Patent Application Serial No. 62/625,560, filed on February 2, 2018.

### FIELD OF THE INVENTION

The present invention is directed to a syringe assembly having a connecting member for connecting to a container for filling and aspirating a syringe. The invention is also directed to syringe assembly having a movable member for modifying the length of the exposed portion of the syringe needle for aspirating and filling the syringe.

### DESCRIPTION OF THE RELATED ART

Needle lengths in the range of 4 mm to 5 mm can be difficult to insert into a container or vial and aspirate due to the short length. The short length requires the needle to pierce a septum in the vial in a straight line to ensure penetration and reduce the risk of the needle bending.

The insertion of a needle into the skin of a patient is determined primarily by the features of the needle and not the features or structure of the needle support as disclosed in Needle Insertion Modeling; Identifiability and Limitations, L. Barbe, Biomedical Signal Processing and Control 2 (207) 191-198. Needle insertion into the skin of patient is generally classified into three phases that influence the injection depth. The first phase corresponds to the initial contact of the needle with the skin where the tissue deforms without puncturing the surface of the skin. A second phase refers to the puncture of the skin and the relaxation of the skin when the insertion force of the needle is stopped. The third phase is where the needle is extracted and pulls or stretches the skin outward as the needle is extracted.

Needle lengths, such as needles having a length of about 4 mm to 5 mm are adapted to inject a medication to a specified target depth in a subcutaneous region. The present invention provides a structure so that a needle can be consistently inserted to a desired target depth. Prior pen needles have the cannula supported on an axial post extending from the hub. The post forms a narrow portion and a relatively wider base that does not contact the skin during the injection. In other pen needles known in the art, a distal face of the hub placed against the injection site may be relatively large, and may be provided with a slight taper at the edge. The edge of the hub can engage the skin when the cannula is inserted at an angle relative to the surface of the skin of the patient.

Various injection devices have been produced where the supporting structure does not contact the skin during injection or extraction of the needle. Other devices have been proposed where the end face of the device is positioned to contact the surface of the skin to limit the depth of penetration into the patient.

Pen-injector delivery devices facilitate self-administration of parenteral medications. Pen needles are a component of needle-based injection systems and consist of a doubled ended cannula assembled into a plastic hub using adhesive. The hub has internal threads, which allow it to be attached to the pen-injector device. Pen needle attachment allows the proximal end of cannula to penetrate through the rubber septum of the medicament cartridge to create the fluid flow path. For many diabetics maintaining blood glucose control is achieved by performing multiple daily injections of insulin into the subcutaneous (SC) tissue using pen injector delivery devices developed to be a convenient, discreet alternative to the vial and syringe. Numerous pen injectors are commercially available in either disposable or multi-use configurations, each offering various patient-centric features. The distal pen needle cannula interfaces with the delivery site providing a conduit for delivery. Pen needle designs are intended to enable consistent delivery to the target tissue space, minimize leakage of injectate, and reduce pain/discomfort and site effects such as bleeding and bruising associated with the injection. The primary design features, needle length/gauge and hub face geometry, in conjunction with mechanics of the delivery system and injection technique, dictate injection success.

Injections may be performed in the intradermal region, the subcutaneous region and the intramuscular (IM) region of the skin. For many types of injectable medications, including insulin, the SC region is preferred for administering an injection. See, for example, Lo Presti, et al., Skin and subcutaneous thickness at injecting sites in children with diabetes: ultrasound findings and recommendations for giving injection, Pediatric Diabetes (2012).

While the prior devices are generally suitable for the intended use, there is a continuing need for improved devices for controlling the depth of penetration of a cannula for delivering a drug or medicament to a selected target area.

EP 1 930 038 A2, EP 3 181 167 A2, EP 2 857 055 A1, GB 2 461 085 A and WO 2006/099441 A2 describe further state of the art relevant for the present invention.

### SUMMARY OF THE INVENTION

The present invention is defined in claims 1 and 9.

The present invention is directed to syringe assembly to assist in aspirating the syringe. The invention in one embodiment is further directed to a syringe assembly that provides the needle with a first exposed length for aspirating the syringe and a second exposed length for injecting the substance to the desire depth into the patient.

The syringe assembly in one embodiment includes a syringe barrel and a needle hub coupled to the distal end of the syringe barrel. A movable connecting member is coupled to the syringe barrel for sliding movement on the syringe barrel. The connecting member slides between a first retracted position where the needle is exposed a length suitable for injecting the substance into the patient and a second position extending at least partially over the needle. The connecting member has a coupling mechanism for coupling with a container or vial containing a substance where the connecting member assists in aspirating the syringe. The syringe barrel and needle slide relative to the connecting member so that the needle is able to pierce of a septum on the container or vial to assist in penetrating the septum in the container and for aspirating the syringe. After aspirating the syringe, the syringe barrel and needle are retracted to remove the needle from the septum and container. The container is then separated from the connecting member and the connecting member is retracted onto the syringe barrel to expose the needle for use.

In one embodiment, the connecting member has a collar at a distal end formed by flexible arms. The arms flex outward to receive the neck of the container so that the container is loaded sideways into the collar relative to a longitudinal axis of the collar. The collar has at least one and typically a plurality of detents to grip the neck of the container.

In another embodiment, the syringe assembly includes a syringe barrel with a sleeve attached to a distal end. A connecting member is mounted on the sleeve for sliding between a retracted position to expose a length of the needle on the syringe, and an extended position covering the needle. The connecting member includes a coupling mechanism for coupling with a container or vial for assisting in aspirating the syringe. The connecting member slides over the neck of the container in an axial direction with respect to the longitudinal axis of the syringe.

In one embodiment, the syringe barrel has a slidable connecting member that slides between a retracted position to expose a length of the needle, and an extended position covering the needle. The connecting member has at least one and typically a plurality of axially extending legs for coupling with a container while aspirating the syringe. The legs include an inwardly extending detent to engage the neck of the container and to align the septum of the container with the needle. The legs are sufficiently flexible to snap over the neck of the container to attach and separate the container from the connecting member. The syringe barrel can also include a sleeve attached to the distal end of the syringe barrel where the connecting member slides on the sleeve.

Another aspect of the syringe assembly is to provide an end cap attached to the syringe barrel that is movable between a first position where the needle extends from the cap a first distance and a second position where the needle extends a second distance. The end cap can have a movable adapter that can pivot over the end cap to cover a portion of the needle. The adapter is moved to the open position to expose the needle having a length suitable to pierce the septum of a container for aspirating the syringe barrel. After filling the syringe barrel, the adapter is moved over the end cap so that the needle has an effective length less than when the cap is open. The cap is positioned to define a stop member to limit the depth of penetration of the needle into the patient.

The features are basically attained by providing a syringe assembly comprising a syringe barrel having a proximal end and distal end, a needle bearing hub coupled to said distal end of the syringe barrel, and a connecting member positioned on the syringe barrel. The connecting member is mounted for sliding between a first position where the needle is exposed and a second position covering the needle. The connecting member is configured for connecting to a container and supporting the container when the needle is advanced to pierce a septum on the container.

The features of the syringe assembly are also provided by a syringe barrel having a proximal end and a distal end, a needle-bearing hub coupled to said distal end of the syringe barrel, and an adapter coupled to the distal end of the syringe barrel. The adapter is movable between a first position to expose a first portion of the needle having a first length, and a second position to expose a second portion of the needle having a second length.

A method is provided for aspirating and filling a syringe assembly and preparing the syringe assembly for use. The method comprises providing a syringe barrel having a proximal end and a distal end, a needle-bearing hub coupled to the distal end of said syringe barrel, and an adapter coupled to the distal end of the syringe barrel. The adapter is movable between a first position to expose a first portion of the needle having a first length, and a second position to expose a second portion of the needle having a second length. The adapter is moved to the first position to expose the needle. The needle pierces a septum on a container for aspirating the syringe. The needle is withdrawn from the container, and the adapter is moved to the second position to expose the second portion of the needle.

These and other features of the invention will become apparent from the following detailed description of the invention, which in conjunction with the drawings disclose various embodiments of the invention.

### BRIEF DESCRIPTION OF THE DRAWINGS

The following is a brief description of the drawings in which:
Fig. 1 is a perspective view of the syringe assembly in one embodiment of the invention;
Fig. 2 is a top perspective view of the syringe assembly of Fig. 1 showing the connecting member in a retracted position;
Fig. 3 is a top perspective view of the syringe assembly in the embodiment of Fig. 1 showing the connecting member in an extended position;
Fig. 4 is a bottom perspective view of the connecting member of Fig. 1;
Fig. 5 is a cross-sectional side view of the connecting member;
Fig. 6 is a bottom view of the connecting member;
Fig. 7 is a partial cross-sectional side view of the connecting member;
Fig. 8 is a bottom perspective view of the needle hub;
Fig. 9 is a cross-sectional view of the needle hub of Fig. 8;
Fig. 10 is a side view showing the connecting member in cross-section where the connecting member is in the extended position;
Fig. 11 is a side view showing the needle penetrating the septum of the container;
Fig. 12 is a perspective view of the syringe assembly in another embodiment;
Fig. 13 is a perspective view of the syringe assembly of Fig. 12 showing the connecting member in a retracted position;
Fig. 14 is a perspective view of the syringe assembly of Fig. 12 showing the connecting member in an extended position;
Fig. 15 is a top perspective view of the connecting member in the embodiment of Fig. 12;
Fig. 16 is a side view of the guide sleeve;
Fig. 17 is a bottom perspective view of the guide sleeve of Fig. 16;
Fig. 18 is a cross-sectional view of the guide sleeve of Fig. 16;
Fig. 19 is a side view with the connecting member in cross-section showing the needle in a retracted position;
Fig. 20 is a side view showing the needle penetrating the septum of the container;
Fig. 21 is a perspective view of the syringe assembly in an exemplary configuration not representing the present invention;
Fig. 22 is a perspective view of the syringe assembly of Fig. 21 showing the connecting member in an extended position;
Fig. 23 is a bottom perspective view of the connecting member of the configuration of Fig. 21;
Fig. 24 is a bottom perspective view in partial cross-section of the connecting member;
Fig. 25 is a side view of the syringe assembly coupled to the container;
Fig. 26 is a side view showing the syringe barrel and connecting member in cross-section;
Fig. 27 is a side view showing the syringe barrel and connecting member in cross-section with the needle penetrating the septum;
Fig. 28 is a perspective view of a syringe assembly in another exemplary configuration not representing the present invention;
Fig. 29 is a bottom perspective view of the connecting member in the configuration of Fig. 28;
Fig. 30 is a cross-sectional view of the connecting member of Fig. 29;
Fig. 31 is a perspective view of the guide sleeve in the configuration of Fig. 28;
Fig. 32 is a cross-sectional view of the guide sleeve of Fig. 31;
Fig. 33 is a perspective view of another exemplary configuration not representing the present invention of the syringe assembly;
Fig. 34 is an exploded perspective view of the syringe assembly of Fig. 33;
Fig. 35 is a perspective view of the syringe assembly of Fig. 33 showing the cap in the open position;
Fig. 36 is a perspective view showing the adapter and cross-section;
Fig. 37 is an enlarged view of the adapter;
Fig. 38 is a front view of the adapter and syringe barrel;
Fig. 39 is a cross-sectional view of the syringe barrel assembly showing the cap in the closed position;
Fig. 40 is a top perspective view of the body of the adapter;
Fig. 41 is a bottom perspective view of the needle hub;
Fig. 42 is a perspective view of the syringe assembly in another exemplary configuration not representing the present invention;
Fig. 43 is an exploded perspective view of the syringe assembly of Fig. 42;
Fig. 44 is a side view of the syringe assembly of Fig. 42;
Fig. 45 is a perspective view of the syringe assembly;
Fig. 46 is an enlarged exploded perspective view of the syringe assembly;
Fig. 47 is a top perspective view of the adapter;
Fig. 48 is a cross-sectional side view of the adapter;
Fig. 49 is a side view of the adapter;
Fig. 50 is a perspective view of the adapter;
Fig. 51 is a perspective view of the syringe assembly in another exemplary configuration not representing the present invention; and
Fig. 52 is a side view of the syringe assembly of Fig. 51.

### DETAILED DESCRIPTION OF THE INVENTION

The syringe assembly of the invention refers to a syringe having a needle or cannula for injecting a medication or other substance into a patient. The terms needle and cannula are used herein interchangeably to refer to a thin tubular member having a sharp end for insertion into an injection site on a subject. A distal direction is in the direction toward the injection end of the syringe assembly, and the proximal direction is the opposite direction. The axial direction refers to a direction along or parallel to the longitudinal axis of the needle and the needle hub and the radial direction refers to a direction perpendicular to the axial direction.

The intradermal layer in adults generally has a thickness of around 2 to 3 mm, so that intradermal injection depth is in a range of up to about 3 mm as measured from the outer surface of the skin. The thickness of the subcutaneous layer varies depending on the age of the patient, gender, body mass index (BMI), and the part of the body where the injection is administered. The subcutaneous region has an average thickness of about 7 mm to about 15 mm. Insulin is preferably delivered to the subcutaneous region.

The syringe assembly is suitable for injections and for injecting a drug to a patient. The above description of the preferred embodiments is not to be deemed as limiting the invention, which is defined by the appended claims. The disclosure is intended to enable the artisan of ordinary skill to practice variants of the invention described without departing from the scope of the invention. Numerical limitations herein, in the specification and in the claims, are understood to be limited by the modifier "about," such that minor departures yielding equivalent results is within the scope of the invention. Features or dependent claim limitations disclosed in connection with one embodiment or independent claim may be combined in another embodiment or with a different independent claim without departing from the scope of the invention.

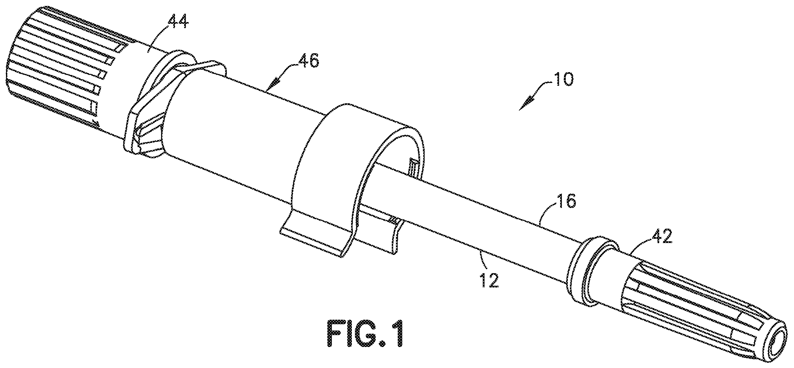

The invention is directed to a syringe assembly that is configured for connecting to a container such as a vial containing a medication to facilitate filling and aspirating syringe. Referring to Figures 1-11, the syringe assembly 10 includes a syringe barrel 12 having a proximal end 14 and a distal end 16. The proximal end 14 receives a movable plunger 18 and stopper for dispensing the substance contained in the syringe assembly.

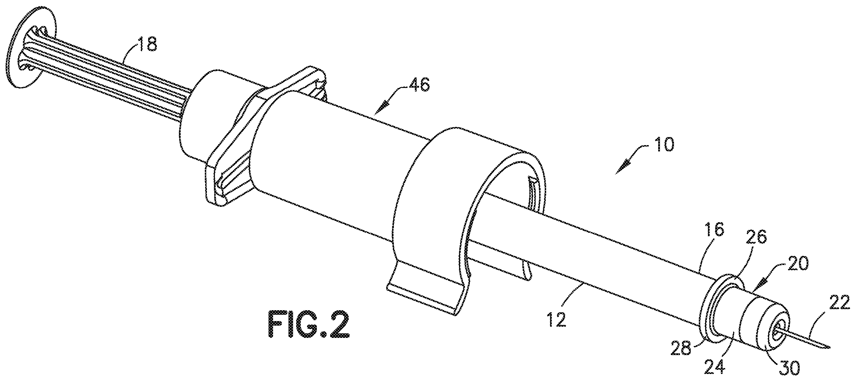

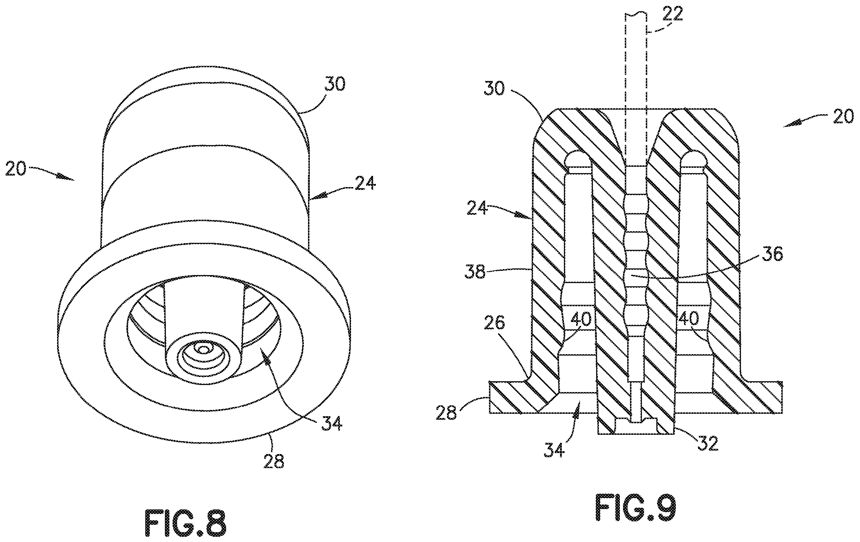

A needle hub 20 is coupled to the distal end 16 of the syringe barrel 12 as shown in Fig. 2. The needle hub 20 shown in Figs. 8 and 9 includes a needle 22 extending axially from the needle hub. Referring to Figs. 8 and 9, the needle hub 20 is configured for coupling to the distal end of the syringe barrel 12. The needle hub 20 has a substantially cylindrical body 24 with a proximal end 26 having outwardly extending flange 28. A distal end 30 projects outwardly for contacting the surface of the skin of the patient during use.

The needle hub 20 has a substantially cylindrical inner post 32 shown in Fig. 9 spaced inwardly from the sidewall 38 of the body 24 to form an annular recess 34. The inner post 32 includes an axial passage 36 for supporting the needle 22. In the embodiment shown, the inner surface of the sidewall 38 of the body 24 includes a plurality of detents forming ribs 40 for coupling to an outer surface of the distal end of syringe barrel 12. The needle 22 in the embodiment shown has a length of about 4-6 mm extending from the distal end 30 of the body 24 although the exposed length of the needle can vary depending on the particular needs of the syringe apparatus.

Referring to Fig. 1, the syringe assembly 10 includes a needle shield 42 coupled to the needle hub 24 covering the needle 22 until ready for use. An end cap 44 is connected to the proximal end of the syringe barrel to cover the plunger 18 during storage.

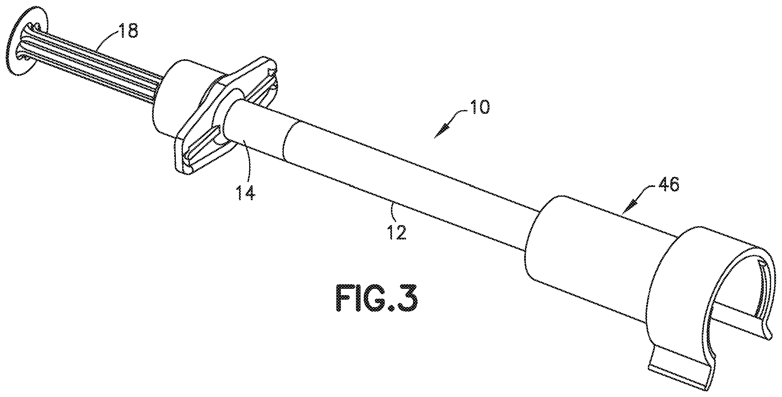

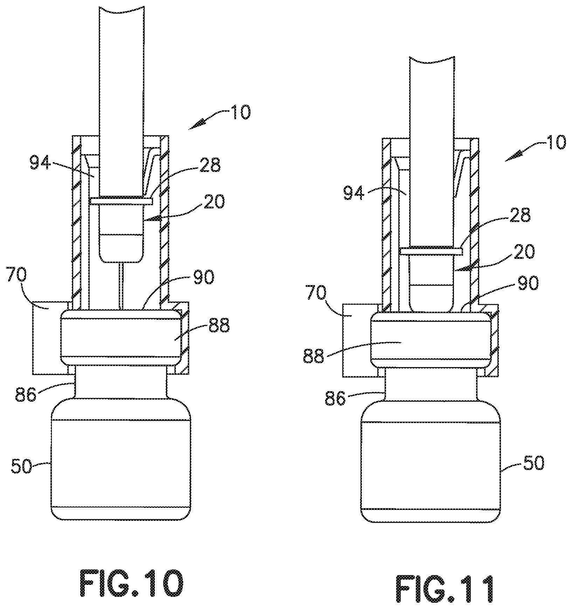

A connecting member 46 is mounted on the syringe barrel 12 for connecting to a container 48 as shown in Fig. 10. In the embodiment shown, the connecting member 46 is mounted on the syringe barrel 12 for sliding movement along the axial length of the syringe barrel. The connecting member can slide between the proximal end of the syringe barrel as shown in Fig. 1 and the distal end of the syringe barrel shown in Fig. 3.

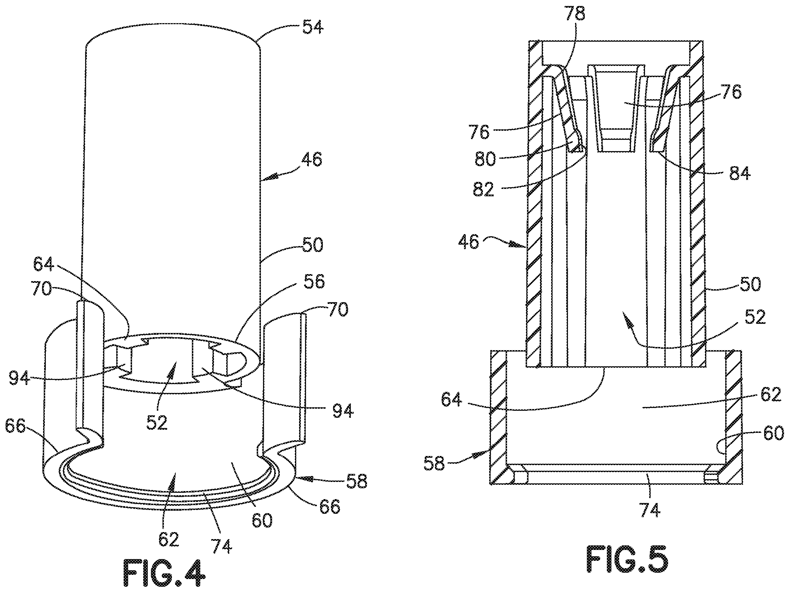

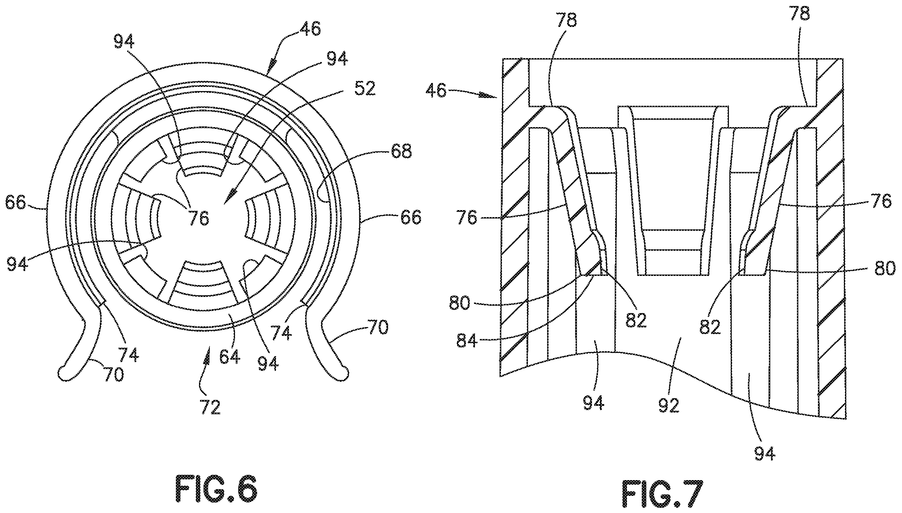

Referring to Fig. 4, the connecting member 46 as a substantially cylindrical body 50 with an axial passage 52 having a dimension for sliding on the outer surface of the syringe barrel. The cylindrical body 50 has a proximal end 54 and a distal end 56. The distal end 56 has a collar 58 extending axially with respect to the longitudinal axis of cylindrical body 50. As shown in Fig. 4 and Fig. 6, the collar 58 has an inner surface 60 forming an axial passage 62 with an inner dimension greater than an outer dimension of the body 50. As shown Fig. 4, the cylindrical body 50 has an axial end face 52 positioned within the axial passage 62. The collar 58 in the embodiment shown is connected directly to the cylindrical body 54 and is a one piece unit. The collar 58 includes two arms 66 with convex inner surfaces 68 shown in Fig. 6 forming the axial passage 62. A free end of the arms 66 include an outwardly flared guide member 70. The free ends of the respective arms 66 are spaced apart a distance to form a side opening 72 into the axial passage 62. A gripping member is provided at a distal end of the collar 58. In the embodiment shown, the gripping member is a detent 74 extending around the inner surface between the free ends of the arms 66. The detent 74 forms an annular rib with a configuration and dimension for connecting to the outer surface of a container while filling and aspirating the syringe assembly. In other embodiments, the gripping member, such as the rib, can be discontinuous to form a plurality of detents.

The proximal end 54 of the cylindrical body 50 includes at least one retaining mechanism for retaining the connecting member on the syringe barrel 12 while allowing sliding movement of the connecting member along the length of the syringe barrel. In the embodiment shown in Fig. 5, the retaining mechanism includes at least one finger 76 projecting into the axial passage 52 of the cylindrical body 50. As shown in Fig. 6, one embodiment includes four fingers 76 that project at an inclined angle relative to the longitudinal axis and extend radially inward toward the center axis and toward the distal end of the cylindrical body 50. The fingers 76 include a first end 78 connected to the inner surface of the cylindrical body 50 and a second free end 80 extending inwardly towards the center axis and toward the distal end of the cylindrical body 50. The fingers 76 are formed at an incline relative to the center axis of the cylinder body so that the free ends contact the outer surface of the syringe barrel as shown in Fig. 10 and Fig. 11. The free ends 80 of the fingers 76 include an inner contact surface 82 for sliding on the outer surface of the syringe barrel. A distal end 84 of the fingers 76 form the stop member to engage the flange of the hub 24 to prevent separation of the connecting member 46 from the distal end of the syringe barrel 12.

The annular inner surface 92 of the body 50 shown in Fig. 7 includes a plurality of ribs 94 extending longitudinally to guide the cylindrical body 50 over the outer surface of the syringe barrel. As shown in Fig. 6, the ribs 94 are oriented between adjacent fingers 76 to stabilize the connecting member on the syringe barrel. The free ends of the fingers 76 project further inward toward the center of the cylindrical body 50 a distance greater than the radial dimension of the ribs 94. As shown in Fig. 10, the ribs 94 extend radially inward a distance to be able to slide on the outer annular surface of the flange 28 of the needle hub 20 and stabilize the sliding movement of the syringe barrel and needle hub within the connecting member 46.

The syringe assembly 10 is assembled initially as shown in Fig. 1. During use, the shield and end cap are removed from the syringe assembly to expose the needle hub and needle as shown in Fig. 2. The connecting member 46 is moved to the distal end of the syringe barrel as shown Fig. 3 where the distal end 84 of the fingers 76 contact the flange 28 of the needle hub 20 as shown in Fig. 10. The container 48 is then connected to the connecting member 46 with the syringe barrel and needle in a retracted position as shown in Fig. 10. The container 48 in the embodiment shown is a vial containing the drug or pharmaceutical to be delivered by the syringe assembly 10 and is snapped onto the connecting member by sliding between the free ends of the arms 66 in a direction perpendicular to the longitudinal axis of the connecting member 46. The container 48 includes a neck 86 having a collar 88 that receives a septum 90 in a typical manner.

The container 48 is connected to the connecting member 46 by sliding the collar 86 of the container laterally through the opening 72 between the free ends of the arms 66. The arms 66 are sufficiently flexible to bend outwardly to allow the collar 88 to slide between the arms and into the open area of the collar 58 of the connecting member 46. As shown in Fig. 10 a bottom edge of the collar 88 of the container 48 is retained by detent 74 of the collar 58 to couple the connecting member 46 to the container 48 and to align the septum and needle. The syringe barrel 12 and the needle 22 slide within the connecting member 46 to enable the needle to pierce the septum 90 where the syringe can be aspirated by manipulating the plunger 18 to fill the syringe barrel. After filling the syringe barrel, the needle 22 is withdrawn from the septum and separated from the container 48. The container 48 can then be removed by sliding laterally through the opening 72 between the flexible arms 66. The connecting member 46 then slides toward the proximal end of the syringe barrel to the position shown in Fig. 2 where the syringe assembly is ready for use.

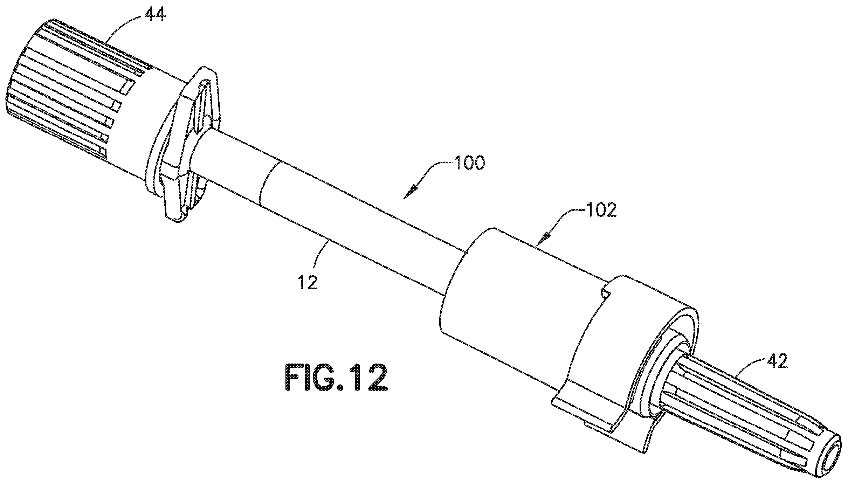

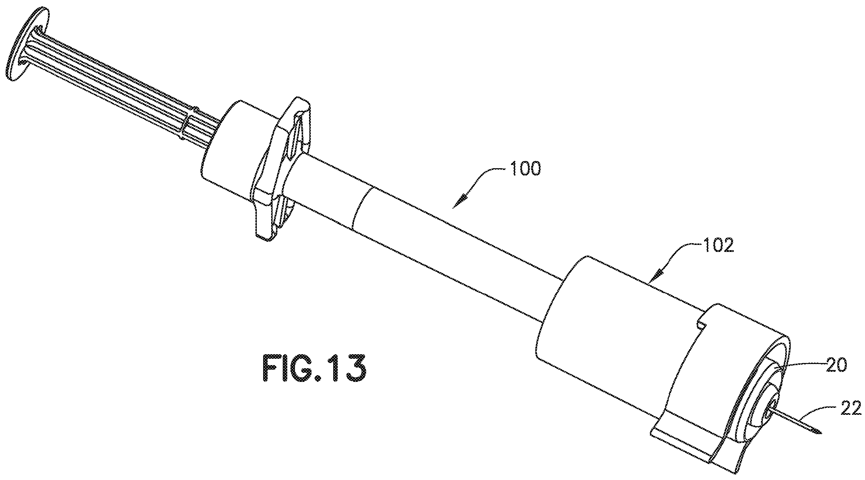

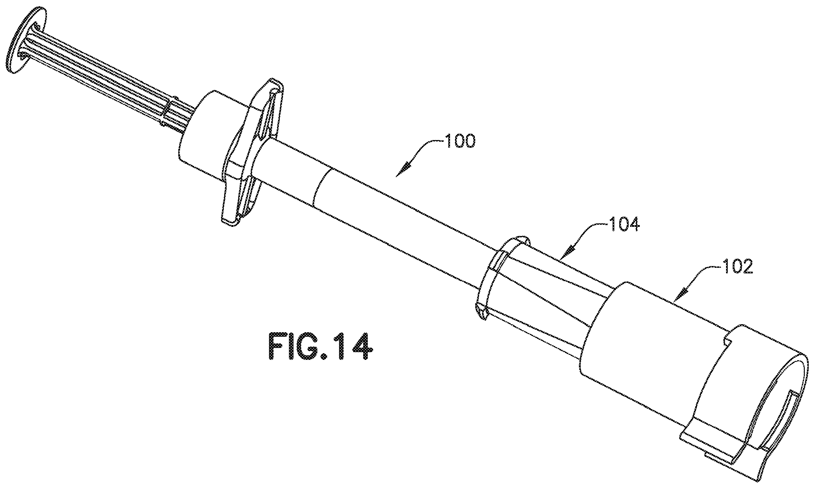

In another embodiment shown in Figs. 12-20 the syringe assembly 100 includes a syringe barrel 12 and needle hub 20 as in the previous embodiment. The syringe assembly 100 and the embodiment shown includes a connecting member 102 that is able to slide from a first position where the needle is exposed to a second position covering the needle 22. A sleeve member 104 is attached to the distal end of the syringe barrel 12 as shown in Fig. 14. The sleeve 104 can be attached to the syringe barrel by a friction fit or interference fit. In the embodiment shown, the distal end 110 of the sleeve 104 is aligned with the distal end of the syringe barrel and/or the needle hub. The connecting member 102 is mounted on the sleeve 104 and slides along the length of the sleeve as shown in Fig. 13 and Fig. 14.

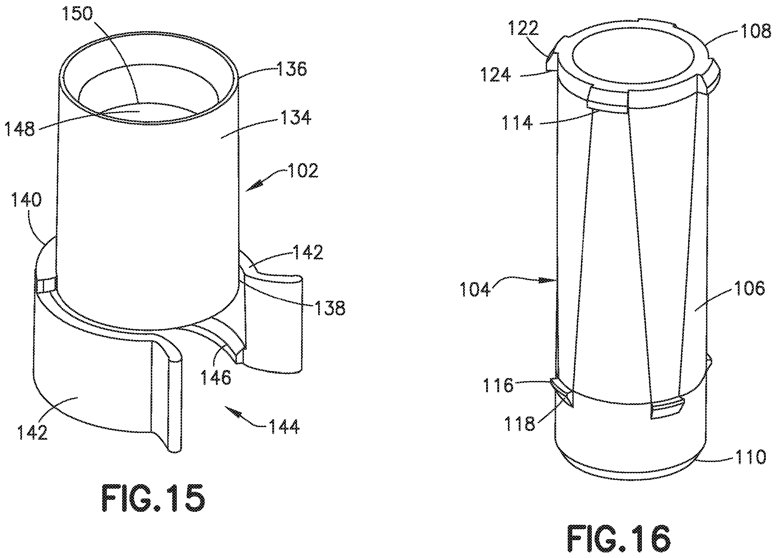

The sleeve 104 as shown in Figs. 16-18 has a generally cylindrical sidewall 106 with an open proximal end 108 and an open distal end 110. The sidewall 106 has an inner surface 112 with a dimension for mating with the outer surface of the syringe barrel 12. As shown in Fig. 16, the proximal end 108 includes a plurality of spaced apart detents 114 extending outwardly for mating with the connecting member 102 and limiting axial movement of the connecting member on the sleeve in the proximal direction. The distal end 110 also includes a plurality of detents 116 spaced around the circumference of the sidewall 106. The detents 116 in the embodiment shown project radially outward and have an inclined outer axial face 118 facing toward the distal end, and a top face 120 extending radially inward and facing in toward the proximal end and the detent 114. The detents 114 have a similar inclined outer axial face 122 facing toward the proximal end, and an inner face 124 extending radially inward toward the distal end and facing the detent 116.

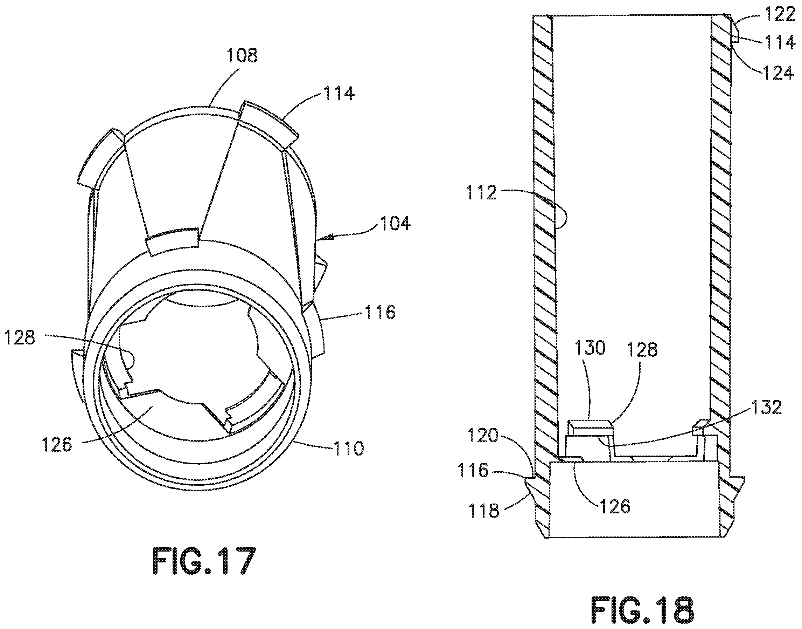

Referring to Figs. 17 and 18, the inner surface 112 of the sidewall 106 includes a coupling mechanism at the distal end for coupling to the flange 28 of the needle hub 20. The coupling mechanism includes a radially inwardly extending lip 126 for engaging a distal side of the flange 28. In the embodiment shown in Fig. 17, three spaced part lips 126 are provided for engaging the flange of the needle hub. An inwardly extending detent 128 is spaced from the lip 126 a distance corresponding substantially to the thickness of the flange 28 of the needle hub 20. In the embodiment shown, the detent 128 has an inclined top surface 130 for sliding over the flange of the needle hub and a straight bottom surface 132 for engaging the top surface of the flange of the needle hub 20 for coupling the sleeve 104 to the needle hub. The sleeve is able to slide over the distal end of the syringe barrel and the needle hub to snap onto the needle hub.

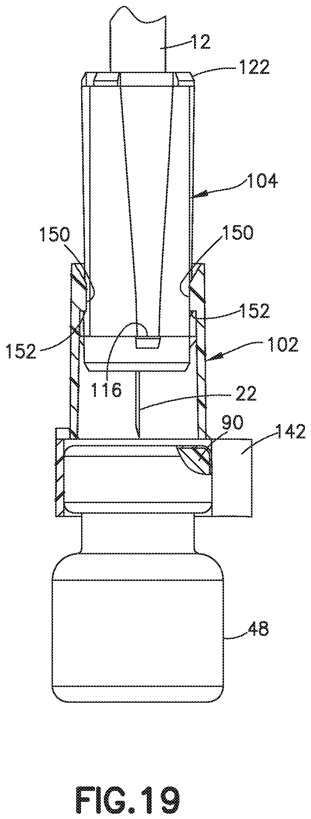

The connecting member 102 is similar to the connecting member of the previous embodiment and includes a cylindrical body 134 with a proximal end 136 and a distal end 138. The distal end 138 includes a collar 140 in a manner similar to the previous embodiment where the collar includes two flexible arms 142. The free ends of the arms 142 are spaced part of distance to form an opening 144 to enable the container 48 to slide laterally into the cavity defined by the collar 140. As in the previous embodiment, a detent 146 is shown as a rib for connecting to the container. The inner surface 148 of the cylindrical body 134 includes an inwardly extending annular flange 150 at the proximal end 136. As shown in Fig. 19, the flange 150 projects radially inward to form a guide surface enabling the connecting member to slide on the sleeve 104. The flange 150 has a bottom face 152 for mating with the detent 116 to limit the sliding movement of the connecting member in the distal direction relative to the sleeve and the syringe barrel as shown in Fig. 19. The flange 150 also engages the detent 122 at the proximal end of the sleeve to limit the sliding movement of the connecting member in the proximal direction relative to the sleeve and the syringe barrel.

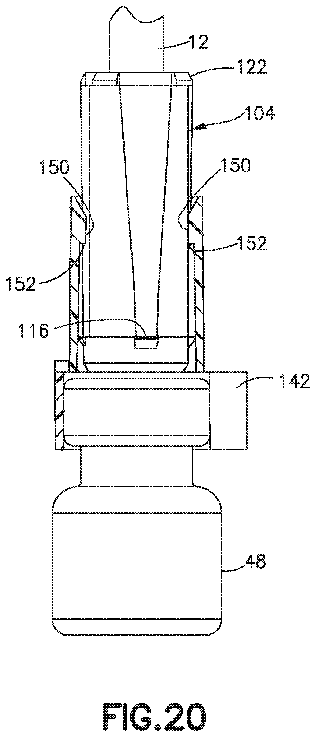

The syringe assembly 100 is used in a manner similar to the previous embodiment. As shown in Fig. 19, the connecting member 102 slides along the sleeve 104 to extend past the end of the sleeve and the needle extending from the needle hub. The container 48 is passed between the flexible arms 142 and captured within the collar 140 by the flexible arms 142 and the detent 146 as shown in Fig. 19. The bottom face 152 of the flange 150 is able to contact the detent 116 to prevent separation of the connecting member 102 from the sleeve 104. The syringe barrel with the sleeve 104 attached is moved toward the container 48 where the needle pierces the septum of the container as shown in Fig. 20. The syringe assembly can then be aspirated and filled with the substance within the container. The syringe barrel and sleeve are then withdrawn to the position shown in Fig. 19 where the needle is separated from the septum in the container. The container can then be removed from the connecting member 102. The connecting member 102 can then slide to the position shown in Fig. 13 where the needle is exposed for injecting the substance to the patient.

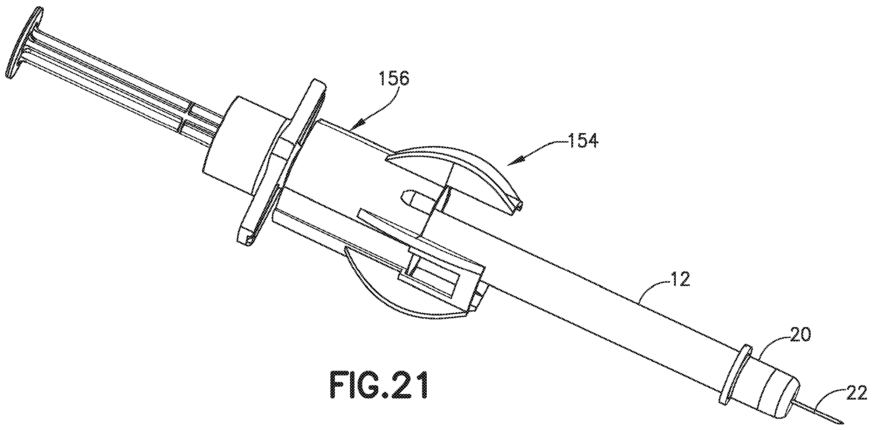

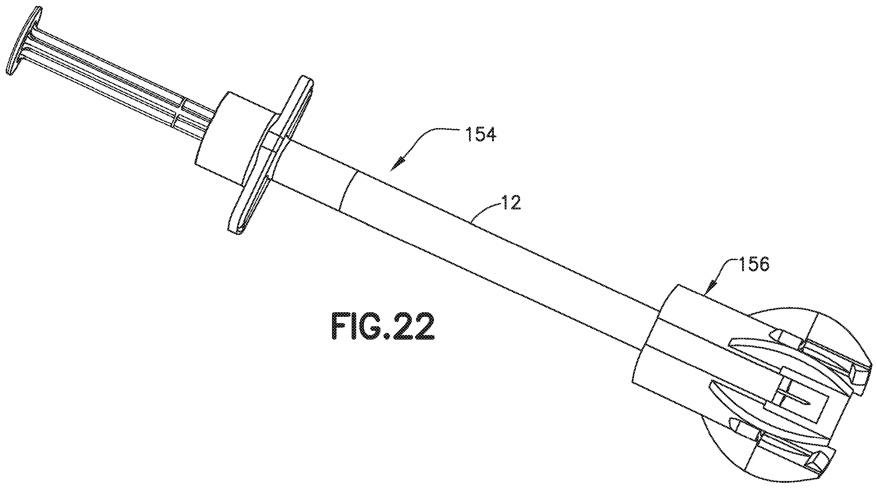

Referring to the configuration of Figs. 21-27, the syringe assembly 154 includes a syringe barrel 12 and a needle hub 20 in a substantially similar manner to the previous embodiments. Therefore, the syringe barrel 12 and needle hub 20 are not discussed in detail in connection with this configuration. A connecting member 156 is provided for sliding on the syringe barrel between a retracted position shown in Fig. 21 to expose the needle 22 and an extended position shown in Fig. 22 for connecting to a container for aspirating the syringe in a manner similar to the previous configurations.

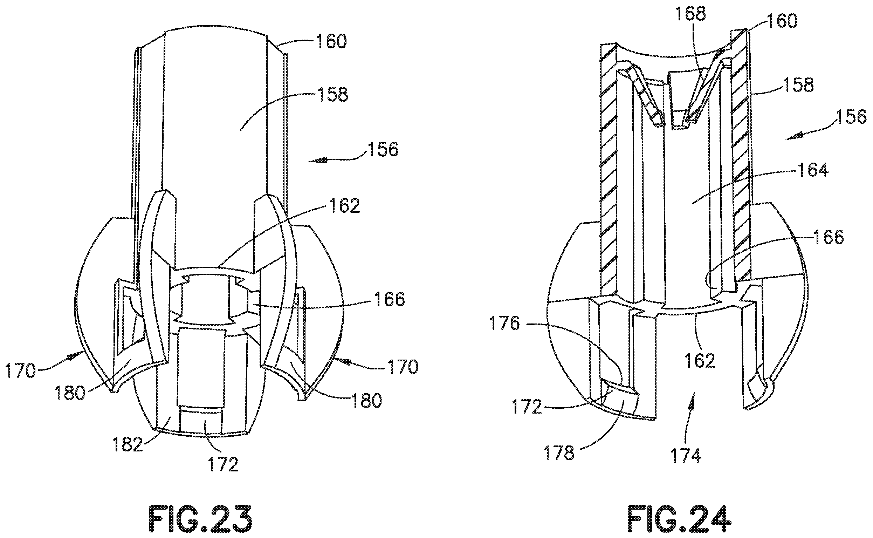

The connecting member 156 includes a cylindrical body 158 shown in Fig. 24 having a proximal end 160 and a distal end 162. As shown in Fig. 24 an inner surface 164 of the body 158 includes a plurality of spaced apart longitudinally extending ribs 166 for guiding the connecting member on the syringe body. The proximal end 160 includes a plurality of retaining members for engaging the flange on the needle hub 20 to limit sliding movement of the connecting member 156 in the distal direction and to resist separation of the connecting member 156 from the syringe barrel. As in the previous embodiment, the retaining members are formed by a plurality of flexible fingers 168 that project at an incline toward the distal end 162 of the body 158 and toward the axial center of the body 158. The free ends of the fingers 168 are oriented for contacting the flange on the needle hub and are sufficiently flexible so that the syringe assembly is assembled by sliding the connecting member onto the distal end of the syringe barrel.

In the configuration shown, the connecting member 156 includes a gripping mechanism at the distal end for coupling with the container 48. The gripping mechanism is formed by a plurality of legs 170 extending axially from the distal end 162 of the body 158. In the configuration shown, the legs 170 are sufficiently flexible to deflect radially outwardly for connecting to the container 48 in an axial direction. The free end of the legs 170 include a detent 172 that projects radially inward into the open area 174 between the legs 170. The detent 172 has an incline top surface 176 and an inclined bottom face 178 for sliding in an axial direction over the collar of the container 48.

In the configuration shown, the legs 170 are formed on the outer surface of the body 158 and have a radial dimension extending radially outward from the body 158 and a longitudinal dimension extending to the free end of the respective leg 170. As shown in Figs. 23 and 24 two adjacent legs 170 are connected by a connecting portion 180 having a convex inner surface 182 complementing the curvature of the container 48. The detent 172 in the configuration shown is formed on the connecting portion 180 and projects inwardly toward the center of the connecting member 156. The connecting portions 180 are spaced around the circumference of the connecting member 156 to form a substantially cylindrical configuration for accommodating the container 48. The legs 170 are sufficiently flexible so that the collar of the container is able to slide axially into the open area 174 by a snap fit where the detents 172 grip the collar of the container and connect to the container to the connecting member 156 by sliding axially into the open area 174. The inclined surfaces 176, 178 of the detents 172 enable the container to slide over the detents for connecting and disconnecting the container to the connecting member 156.

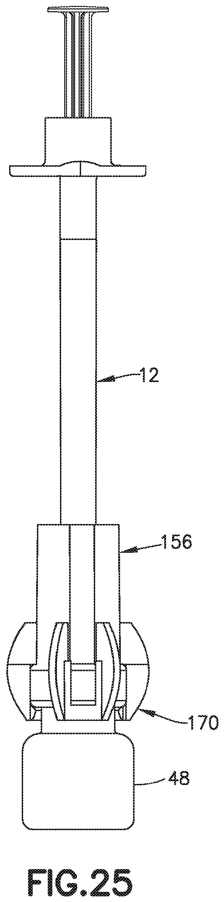

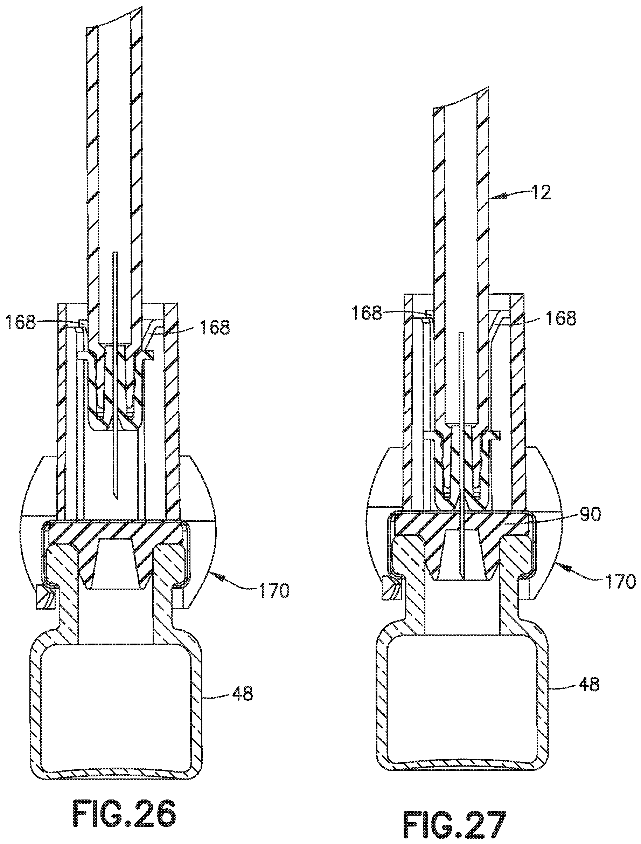

Referring to Fig. 25 and Fig. 26 the connecting member 156 is moved to the distal end of the syringe barrel and the container 48 is snapped onto the flexible legs 170 of the connecting member 156. As shown in Fig. 27 the syringe barrel and needle slide toward the distal end of the connecting member 156 so that the needle pierces the septum of the container 48. The plunger can then be actuated to withdraw the substance from the container and aspirate the syringe. The syringe barrel and needle are withdrawn from the septum in the container. The container is then separated from the connecting member 156. The connecting member 156 can then slide toward the proximal end of the syringe barrel as shown in Fig. 21 to expose the needle for injecting the substance into the patient.

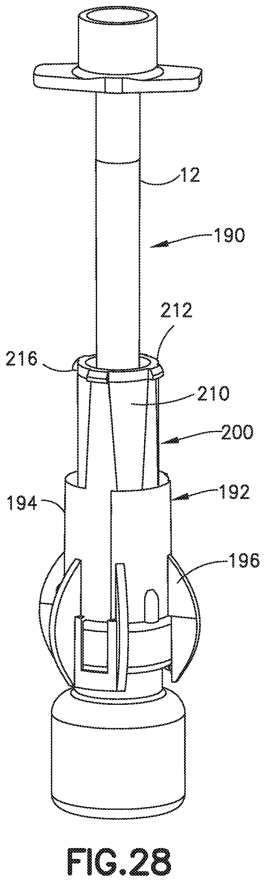

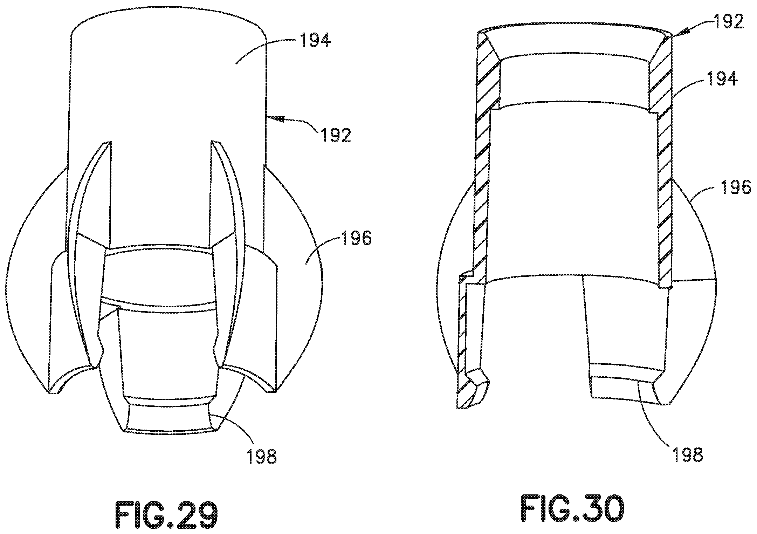

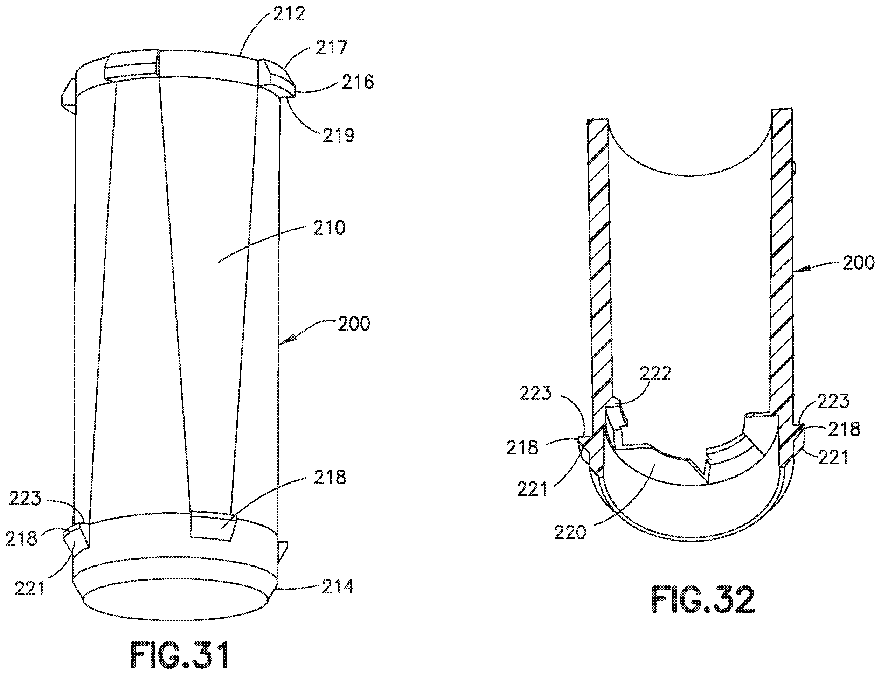

Figs. 28-32 show a further configuration of a syringe assembly 190 similar to the previous embodiment. The syringe assembly 190 includes a syringe barrel 12 and a needle hub 20 as in the previous configuration. A connecting member 192 is mounted on the syringe barrel for connecting to the container in a manner similar to the configuration of Figs. 21-27. The outer surface of the body 194 of the connecting member 192 includes flexible legs 196 for snapping over the end of the container for coupling the container to the connecting member as in the previous embodiment. The flexible legs 196 include the detent 198 projecting radially inward for gripping the collar of the container.

A sleeve 200 is coupled to the distal end of the syringe barrel as in the configuration of Fig. 12. The sleeve 200 as a substantially cylindrical body with a proximal end 212 and a distal end 214. As in the previous embodiment, the proximal end 212 includes a plurality of outwardly extending detents 216 having an inclined proximal face 217 and a flat distal face 219 for limiting sliding movement of the connecting member 192 in the proximal direction. The distal end 214 also includes a plurality of detents 218 projecting radially outward for capturing the connecting member while allowing the connecting member to slide along the longitudinal dimension of the sleeve 200 and the syringe barrel. The detents 218 include an inclined distal face 221 and a flat proximal face 223 for limiting sliding movement of the connecting member in the distal direction relative to the sleeve. The inner surface of the body 210 includes an inwardly extending lip 220 and a spaced apart detent 222 for capturing the flange of the needle hub for coupling the sleeve to the syringe barrel and needle hub.

As in the previous embodiments, the connecting member 192 slides toward the distal end of the syringe barrel and over the sleeve 200 where the legs 196 are able to receive the container and couple the container to the connecting member 192. As in the previous embodiment, the legs slide axially over the end of the container. The syringe barrel with the sleeve attached then slide relative to the connecting member 192 so that the needle pierces the septum of the container for filling and aspirating the syringe barrel. The needle is withdrawn from the septum after filling and the container is separated from the connecting member 192. The connecting member 192 then slides toward the proximal end of the syringe barrel to expose the needle for injecting the substance to the patient.

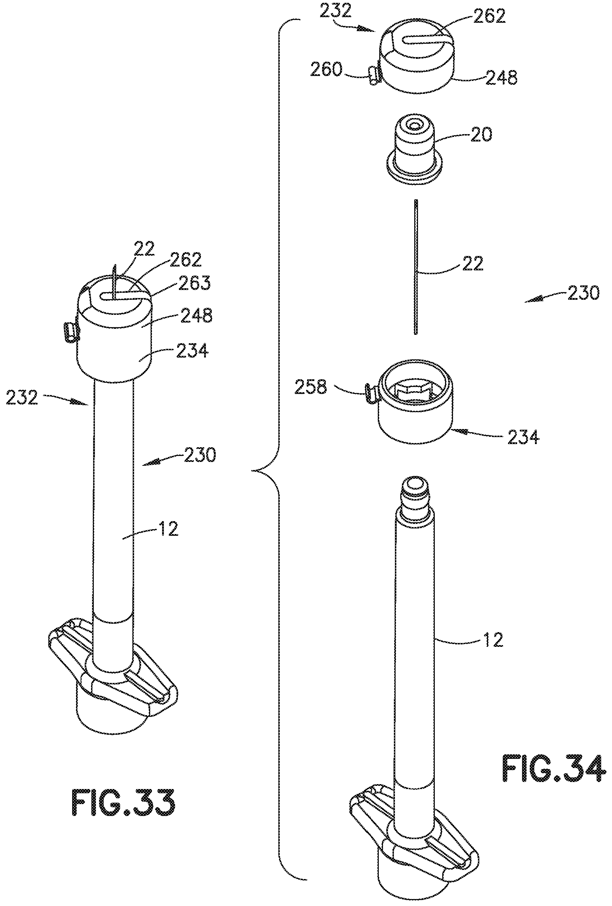

Another configuration of the syringe assembly is shown in Figs. 33-41. The syringe assembly 230 in the configuration shown is configured so that the needle 22 extends from the end of the syringe and has a length to assist in piercing the septum of the container for filling and aspirating the syringe and where the syringe assembly limits the exposed length of the needle to control the depth of penetration into the skin of the patient.

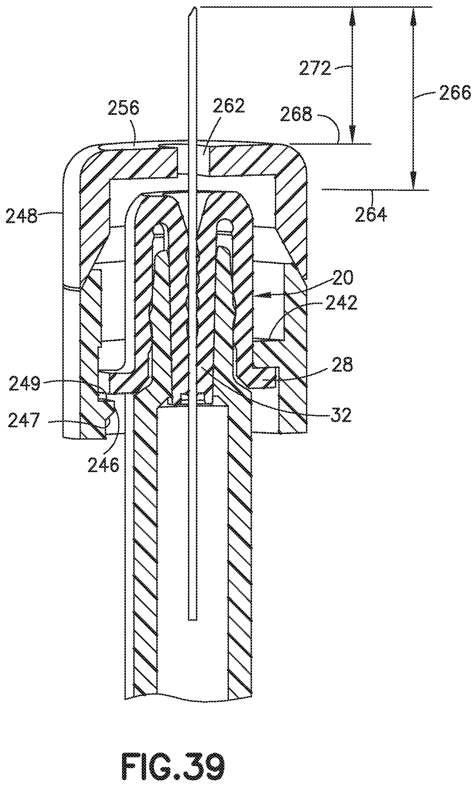

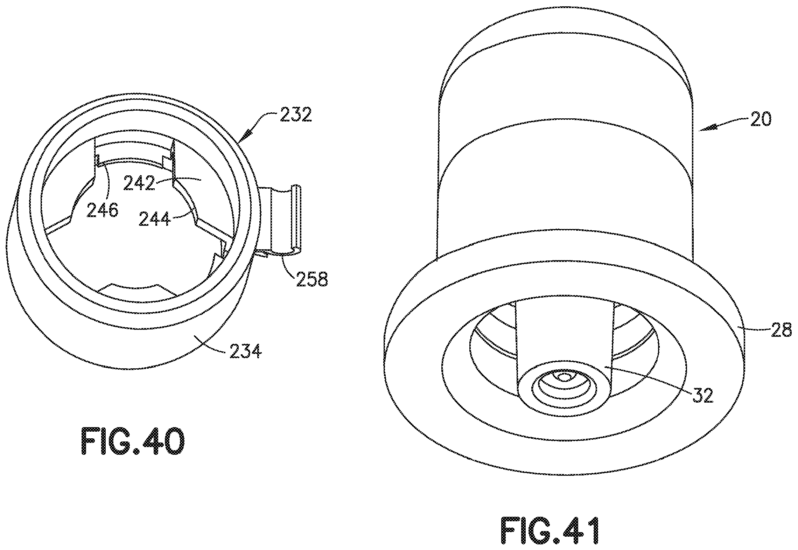

The syringe assembly 230 includes the syringe barrel 12 and the needle hub 20 shown in Fig. 41 coupled to the tip at the distal end of the syringe barrel as shown in Fig. 39. An adapter 232 is coupled to the distal end of the syringe barrel and the needle hub. As shown in Fig. 39, the needle hub 20 supports the needle 22 for coupling the needle to the syringe barrel. The needle hub 20 shown in Fig. 41 as in the previous configuration includes an outwardly extending radial flange 28. The inner post 32 is received in the outlet end of the tip of the syringe barrel as shown in Fig. 39.

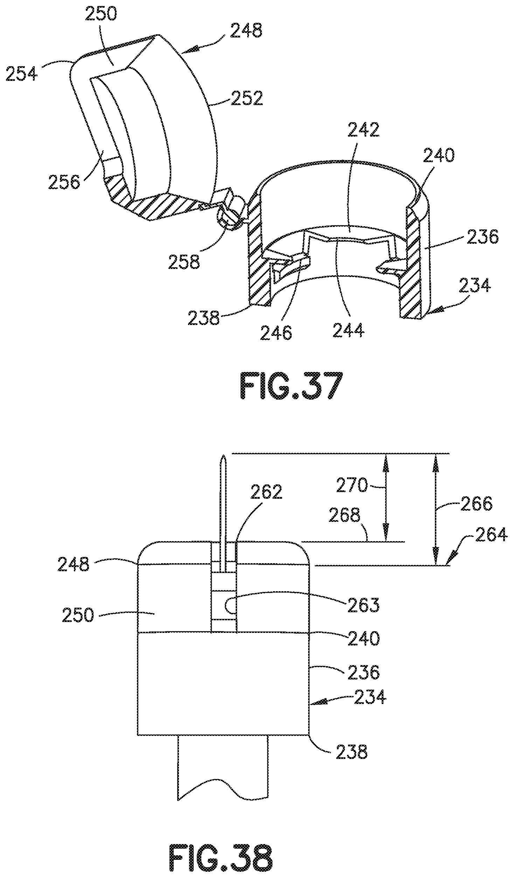

The adapter 232 includes a body 234 for coupling to the flange 28 of the needle hub 20 shown in Figs. 33-36. Referring to Figs. 37-40, the body 234 has a substantially cylindrical sidewall 236 with an inner dimension complementing the outer dimension of the syringe barrel and needle hub. The body 234 has an open proximal end 238 and an open distal end 240 shown in Fig. 37. As shown in Fig. 40, an inwardly extending lip 242 projects radially inward at the proximal end of the body 234. In the configuration shown, three lips 242 are spaced apart around the circumference of the inner surface of the sidewall 236. As shown in Fig. 37, the lips 242 have a concave shaped inner surface complementing the outer surface of the needle hub and have a dimension for mating with the inner face of the flange 28. The detent 246 is spaced from the lips 242 toward the proximal end 238. The detent 246 is spaced from the lips 242 a distance corresponding substantially to the thickness of the flange 28 for coupling the body 234 to the needle hub. As shown in Fig. 39, the detents 246 have an inclined proximal face 247 and a flat distal face 249.

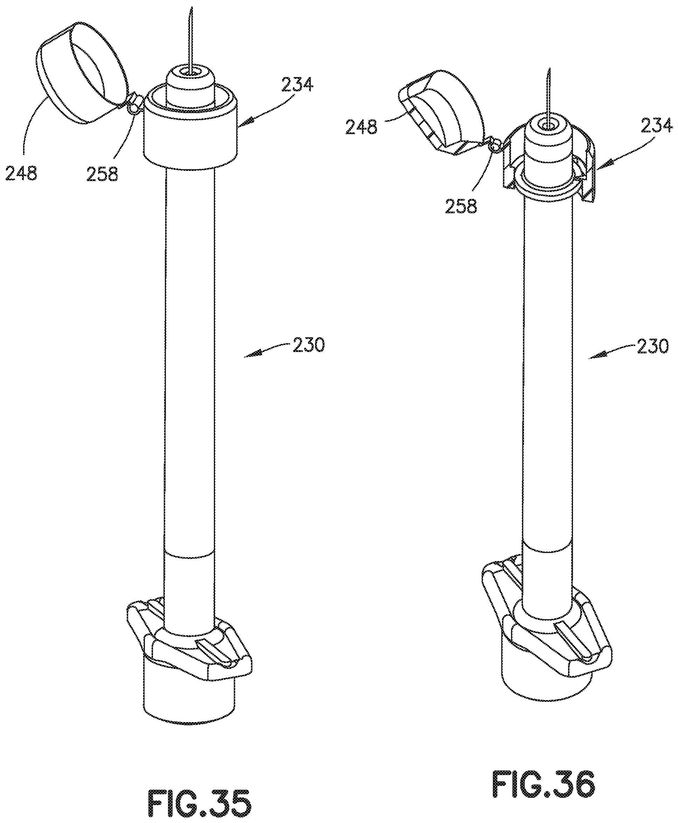

As shown in Fig. 35 and Fig. 36, the body 234 has an axial length less than the axial length of the needle hub so that the axial face of the needle hub and needle extend from the open distal end 240. A cap member 248 is coupled to the body 234 as shown in Fig. 39. In the configuration shown, the cap member 248 has a substantially cylindrical shape corresponding to the shape and outer dimension of the body 234. The cap member 248 has a substantially cylindrical sidewall 250 with an open proximal and 252 and a distal end 254 shown in Fig. 37. An end wall 256 is provided at the distal end 254 to close the distal end of the cap 248. The cap member 248 is configured for connecting to the body 234 so that the needle projects through the cap member 248 as shown in Fig. 38.

The cap member 248 is configured for covering a portion of the needle during injection of the substance into the patient and exposing a second portion of the needle during filling and aspiration of the syringe. The needle in this configuration is a short needle having an axial length of about 4 to 6 mm. Needles of this length can be difficult to pierce the septum of a container to fill and aspirate the syringe. The cap member 248 can be separated from the body 234 to expose the end of the needle hub and the entire length of the portion of the needle extending from the needle hub. With the entire length of the needle exposed, the needle has an effective length to pierce the septum of the container for filling and aspirating the syringe. Under some circumstances, the length of the needle may be greater than the desired depth of penetration during use. The cap member can be attached to the body 234 to reduce the exposed length of the needle to a desired length.

In the configuration shown in Figs. 35-37, the body 234 includes a hinge 258 that mates with a hinge member 260 on the cap member 248 to enable the cap member to pivot between an open position shown in Fig. 35 and a closed position shown in Fig. 33 and Fig. 38. The cap member 248 includes an opening 262 in the top wall 256 to allow the needle to extend through the cap member 248 and beyond the top wall 256. In the configuration shown, the opening 262 in the cap member 248 is in the form of a slot 263 extending along the sidewall 250 and the end wall 256 to enable the cap member to pivot about the axis of the hinge assembly without interference with the needle.

Referring to Figs. 38 and 39 the axial end of the needle hub is oriented in a plane indicated by reference number 264 where the needle 22 extends a first length indicated by arrow 266. By connecting the cap member 248 to the body 234 the exposed effective length of the needle is less than when the cap member is removed. The end wall 256 of the cap member 258 as shown in Figs. 38 and 39 lying in a plane 268 defines a length of the needle indicated by arrow 270. In one configuration, the needle can extend from the needle hub and have an exposed length of about 6 mm to enable the needle to pierce the septum of the container for filling and aspirating the syringe. During use, the cap member 248 is positioned to cover the end of the body 234 and the end of the needle hub to shorten the exposed length of the needle. In one configuration, the exposed length of the needle extending from the end wall 256 of the cap member 248 is about 4 mm. The length of the needle and the dimension of the cap member 248 can be modified to provide a desired exposed length of the needle for penetrating the skin to a desired depth.

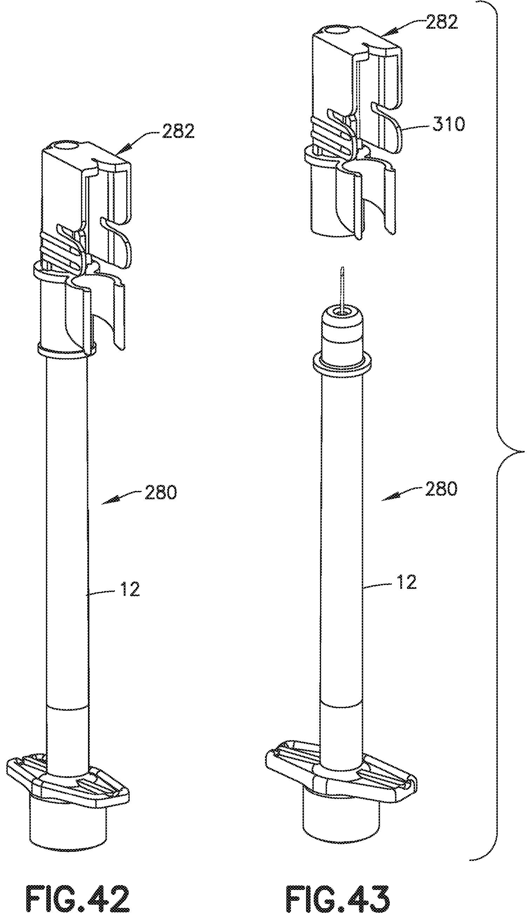

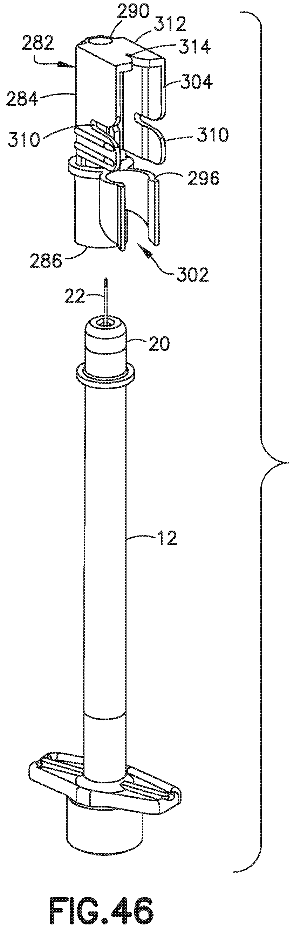

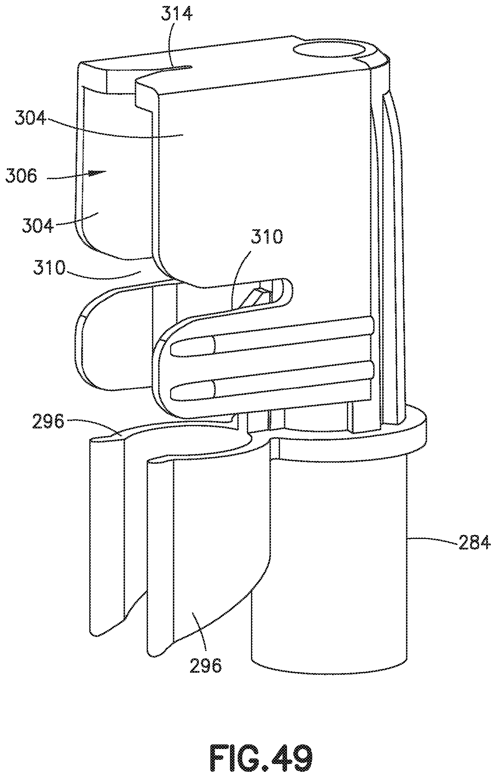

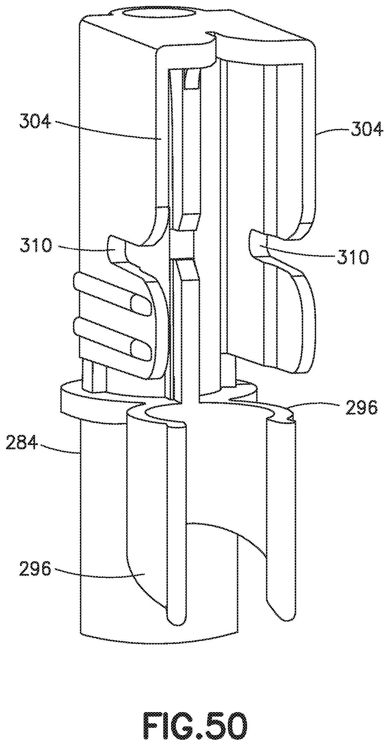

In a further configuration shown in Figs. 42-50, the syringe assembly 280 includes an adapter shown as a needle shield 282 that is capable of covering the needle prior to use and can be attached to the syringe barrel to expose a selected length of the needle projecting from the needle shield. The syringe assembly 280 includes a syringe barrel 12 and a needle hub 20 as in the previous configurations.

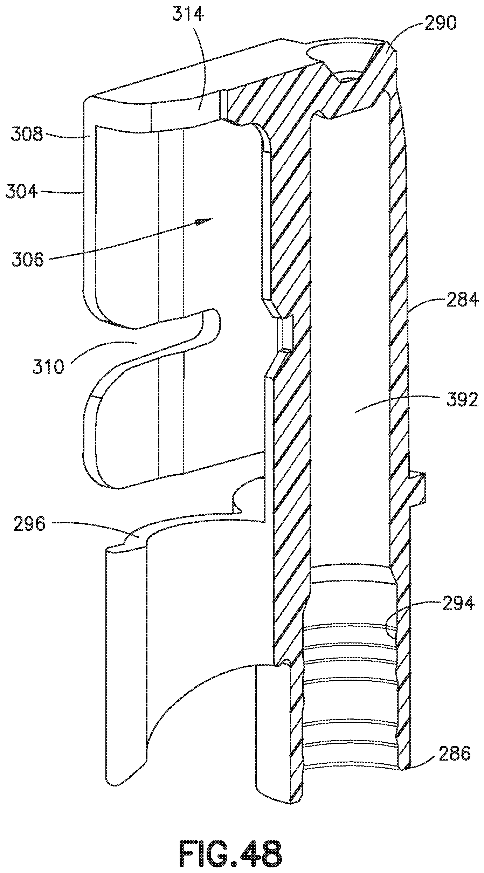

The needle shield 282 has a body 284 with an open proximal end 286 and a close distal end 290 forming an axially extending cavity 292 shown in Fig. 48. The cavity 292 has an axial length complementing the length of the needle hub 20 and the needle 22. An inner surface of the cavity 292 includes inwardly extending annular ridges 294 for gripping the outer surface of the needle hub. As shown in Fig. 42 the shield 282 is position on the needle hub with the needle hub and the needle extending into the cavity 292. During use, the needle shield 282 is removed from the needle hub to expose the needle so that the needle can pierce the septum of the container to fill and aspirate the syringe.

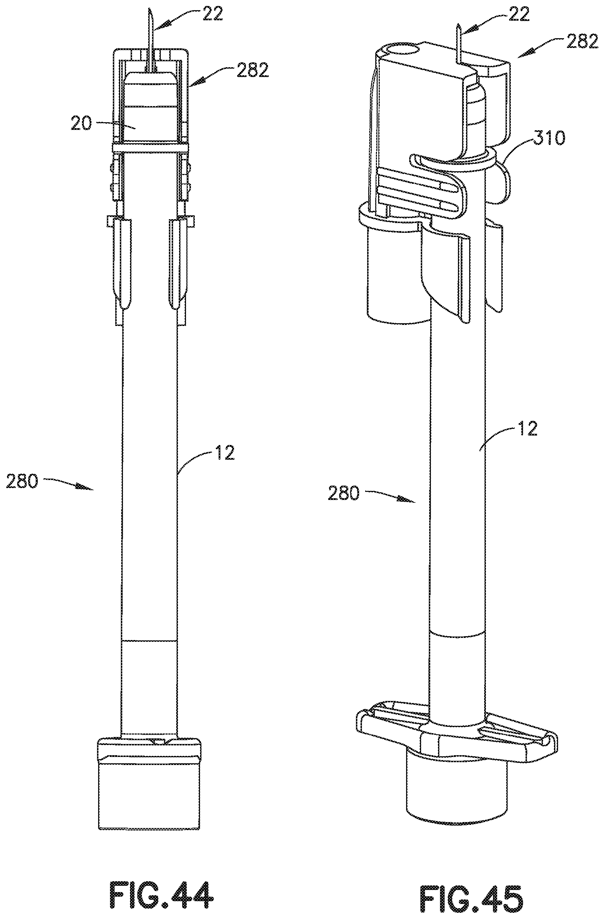

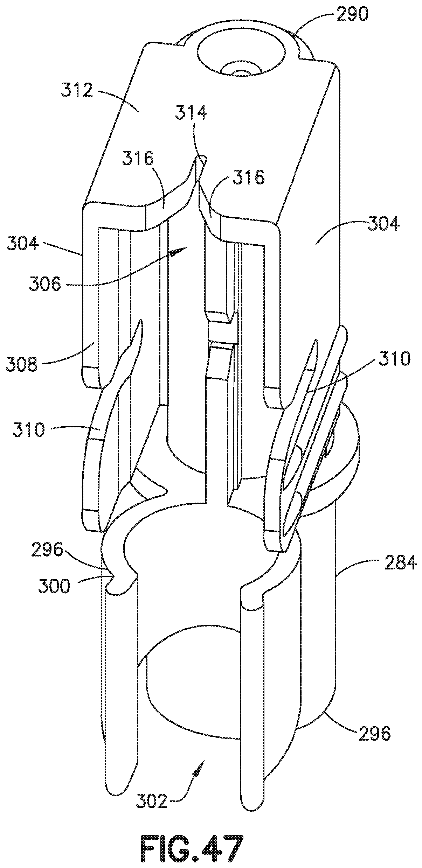

The needle shield 282 also forms a limiter to limit the axial length of the exposed portion of the needle during the injection. Referring to Figs. 44, 45, and 47-50, the needle shield 282 includes a pair of arms 296 extending outwardly from the body 248. The arms 296 have a curvature corresponding to the curvature of the outer surface of the syringe barrel for snapping laterally onto the syringe barrel. The arms 296 have the concave inner surface 298 with free ends 300 shown in Fig. 47 spaced part a distance to define an opening 302. The arms 296 are sufficiently flexible so that the free ends 300 of the arms are able to slide sideways over the outer surface of the syringe barrel to attach the needle shield 282 to the syringe barrel.

The body 284 includes parallel, spaced apart side walls 304 forming a cavity 306 with a dimension for receiving the needle hub. The sidewalls 304 have an open side 308 so that the syringe and needle hub can slide laterally into the cavity 306. The sidewalls 304 include a slot 310 for accommodating the flange 28 on the needle hub 20 as shown in Fig. 45. An end wall 312 extending between the sidewalls 304 includes a slot 314 for accommodating the needle 22 as shown in Fig. 45. The slot 314 is formed by flared edges 316 to assist in guiding the needle into the slot 314.

During use, the needle shield 282 is separated from the syringe to expose the needle and the needle hub. The syringe can then be filled by the needle piercing the septum of the container in the usual manner. The exposed length of the needle with the needle shield removed is sufficient to pierce the septum for filling the syringe. The needle shield 282 can then be snapped onto the distal end of the syringe barrel with the flange of the needle hub positioned in the slot 310 so that the end wall 312 is spaced from the distal end of the needle hub thereby shortening the effective length of the needle. The end wall 312 forms a limiting contact surface to limit the depth of penetration into the patient during use. The location of the slot 310 relative to the end wall 312 determines the effective reduction in the exposed length of the needle. In one configuration more than one slot can be provided in the sidewalls 304 to provide a selected exposed length of the needle during use.

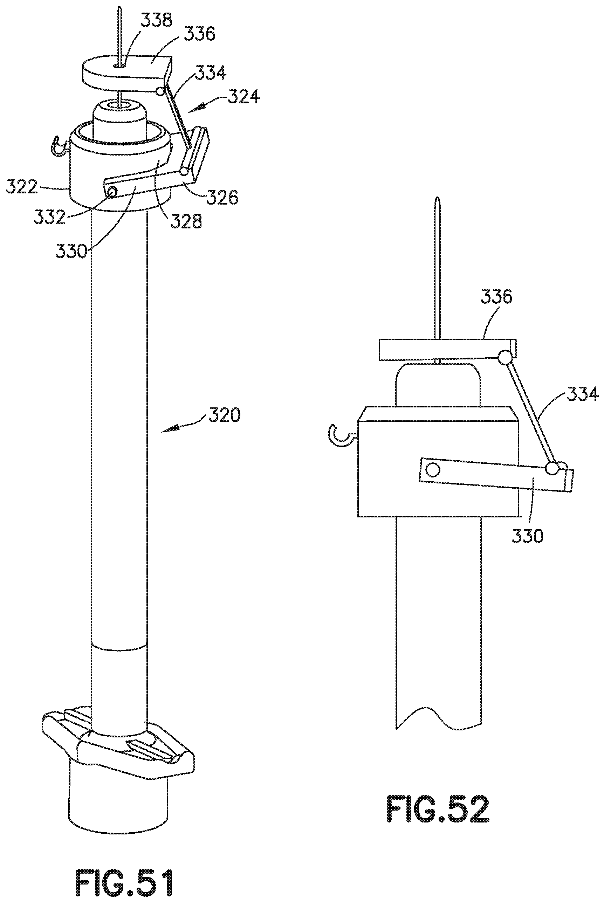

In another configuration shown in Figs. 51 and 52, an adapter 320 is coupled to the distal end of the syringe barrel in a manner similar to the previous configuration. The adapter 320 includes a body 322 that is coupled to the distal end of the syringe barrel 12 and the needle hub 20. The body 322 in the configuration shown has a substantially cylindrical shape and is provided with inwardly extending detents in a manner similar to the configuration shown in Fig. 39 for coupling to the flange of the needle hub. As in the previous configuration, the axial end of the needle hub extends past the distal end of the body 322 and the needle 22 extends from the distal end of the needle hub 20.

A depth limiting assembly 324 is coupled to the body 322 that can be moved between a first position overlying the axial end of the needle hub and a second position exposing the axial end of the needle hub. The assembly 324 includes an arm 326 pivotally connected to the body 322. In the configuration shown, the arm 326 includes a U-shaped recess 328 to accommodate the outer dimension of the body 322. The U-shaped recess 328 forms projections 330 where the ends of the projections are pivotally connected to the body 322 by a pivot pin 332. A connecting arm 334 has a first end pivotally connected to the arm 326 by a pivot pin 327 for pivoting freely with respect to the arm 326. A second end of the connecting arm 334 is pivotally connected to a shield 336 by a pivot pin 329. The shield 336 is provided with an opening 338 with a dimension sufficient to allow the needle to pass through.

During use, the arm 326 can be pivoted toward the distal end of the body 322 a distance so that the shield 336 can slide from the needle. The shield 336 can then pivot away from the needle to expose the full length of the needle extending from the needle hub. During use, the shield 336 can be pivoted back to a position where the needle extends through the opening 338. The arm 326 and the shield 36 move to a position where the shield 336 contacts the axial face of the needle hub shown in Fig. 52. The thickness of the shield 336 shortens the effective exposed length of the needle to define the length of the needle for penetrating the skin of the patient to a desired depth.

In another configuration, a depth limiting assembly on the distal end of the syringe barrel is able to slide from a retracted position to an extended position to adjust the exposed length of the needle. The depth limiting assembly in this configuration can be, for example, a block member that is able to slide on the needle or a portion of the syringe. The block can be positioned on or over the needle and positioned against the distal end of the syringe so that the needle extends past the block a first distance suitable for piercing the septum on the container to aspirate the syringe. By way of example, the needle can have an exposed length of about 6 mm when the block is positioned against the distal end of the syringe. The block is movable by sliding on the needle and/or the syringe to a second position to shorten the exposed end of the needle for use in injecting the patient. A suitable mechanical mechanism is typically provided to control the movement of the block and fix the position of the block relative to the needle. In one configuration, the mechanical mechanism can be scissors type arrangement that can move the block between a retracted position against the distal end of the syringe and an extended position to shorten the effective or exposed length of the needle that is less than when the block is in the first position. The scissors mechanism can include two arms having one end connected together and the opposite end pivotally connected to one of the syringe or the movable block. In an alternative configuration, the mechanical mechanism can be a threaded coupling between the movable block and the syringe.

The foregoing embodiments and advantages are exemplary and are not intended to be construed as limiting the scope of the invention. The description of alternative embodiments are intended to be illustrative, and not to limit the scope of the present invention. Various modifications, alternatives, and variations will be apparent to those skilled in the art, and are intended to fall within the scope of the invention. It is particularly noted that the features of different embodiments and claims may be combined with each other as long as they do not contradict each other. Accordingly all such modifications are intended to be included within the scope of this invention as defined in the appended claims and their equivalents.

## Claims

1. A syringe assembly comprising:
a syringe barrel (12) having a proximal end (14) and a distal end (16), a needle hub coupled to said distal end of said syringe barrel; and
a sleeve (104, 200) attached to the syringe barrel, said sleeve having a distal end aligned with the distal end of the syringe barrel;
**characterized in that** the syringe assembly further comprising:
a connecting member (102, 156), said connecting member has a cylindrical shape and is slidably mounted on said sleeve for sliding between a first position where said needle is exposed and a second position covering said needle, said connecting member having a proximal end having internal retaining members for retaining said connecting member on said sleeve and said syringe barrel, and a distal end having a coupling mechanism (74, 126) for coupling with a container, wherein said retaining member on said distal end of said connecting member includes an inwardly extending detent (74, 146) configured for engaging the container and supporting the container when said syringe barrel and needle move relative to said connecting member to pierce a septum (90) on said container.

2. The syringe assembly of claim 1, wherein said connecting member has a cylindrical shape and is movable relative to said syringe barrel.

3. The syringe assembly of claim 2, wherein said connecting member includes a collar (58) at said distal end, said collar having an open side with a dimension for receiving the container to slide laterally into said connecting member, and where said detent is provided on an inner surface of said collar.

4. The syringe assembly of claim 3, wherein said sleeve is positioned on said distal end of said syringe barrel, and where said sleeve has an outwardly extending detent on a proximal end of said sleeve and an outwardly extending detent of a distal end of said sleeve for limiting axial movement of connecting member on said sleeve.

5. The syringe assembly of claim 1, wherein said coupling mechanism of said connecting member includes a plurality of gripping members (170) extending axially from said distal end of said connecting member.

6. The syringe assembly of claim 5, wherein said gripping members are spaced around said connecting member and where each of said gripping members include an inwardly extending detent for coupling to the container.

7. The syringe assembly of claim 6, wherein said gripping members are flexible and bend radially outward to couple with the container.

8. The syringe assembly of claim 4, wherein said sleeve has an inner surface with a first detent (220) and a second detent (222) to retain said sleeve on said distal end of said syringe barrel, and where said connecting member is slidably mounted on said sleeve.

9. A method for aspirating and filling a syringe, comprising
providing a syringe assembly according to claim 1;
connecting the container to said connecting member (102, 156) and piercing the septum (90) on said container with said needle and filling said syringe barrel (12);
separating said container from said connecting member (102, 156) and retracting said connecting member (102, 156) to expose said needle.

10. The method of claim 9, wherein said connecting member (102, 156) is mounting on said syringe barrel (12), said method comprising;
sliding the connecting member (102, 156) from a retracted position where said needle is exposed to an extended position covering said needle;
coupling said connecting member (102, 156) to the container and sliding said syringe barrel (12) with respect to said connecting member (102, 156) whereby said needle pierces the septum (90) on the container.

## Patentansprüche

1. Spritzenanordnung, umfassend:
einen Spritzenzylinder (12) mit einem proximalen Ende (14) und einem distalen Ende (16), einen Nadelansatz, der mit dem distalen Ende des Spritzenzylinders gekoppelt ist; und
eine an dem Spritzenzylinder angebrachte Hülse (104, 200), wobei die Hülse ein distales Ende aufweist, das mit dem distalen Ende des Spritzenzylinders ausgerichtet ist;
**dadurch gekennzeichnet, dass** die Spritzenanordnung ferner umfasst:
ein Verbindungselement (102, 156), wobei das Verbindungselement eine zylindrische Form aufweist und verschiebbar an der Hülse angebracht ist, um zwischen einer ersten Position, in der die Nadel freiliegt, und einer zweiten Position, in der die Nadel abgedeckt ist, verschoben zu werden, wobei das Verbindungselement ein proximales Ende mit inneren Rückhalteelementen zum Zurückhalten des Verbindungselements an der Hülse und an dem Spritzenzylinder und ein distales Ende mit einem Kopplungsmechanismus (74, 126) zum Koppeln mit einem Behälter aufweist, wobei das Rückhalteelement am distalen Ende des Verbindungselements eine sich nach innen erstreckende Rastung (74, 146) umfasst, die zum Eingreifen am Behälter und zum Halten des Behälters ausgebildet ist, wenn sich der Spritzenzylinder und die Nadel relativ zum Verbindungselement bewegen, um ein Septum (90) an dem Behälter zu durchstechen.

2. Spritzenanordnung nach Anspruch 1, wobei das Verbindungselement eine zylindrische Form aufweist und relativ zum Spritzenzylinder bewegbar ist.

3. Spritzenanordnung nach Anspruch 2, wobei das Verbindungselement an dem distalen Ende einen Kragen (58) umfasst, wobei der Kragen eine offene Seite mit einer Abmessung zum Aufnehmen des Behälters aufweist, sodass der Behälter seitlich in das Verbindungselement gleiten kann, und wobei die Rastung an einer Innenfläche des Kragens vorgesehen ist.

4. Spritzenanordnung nach Anspruch 3, wobei die Hülse an dem distalen Ende des Spritzenzylinders positioniert ist und wobei die Hülse an einem proximalen Ende der Hülse eine sich nach außen erstreckende Rastung und an einem distalen Ende der Hülse eine sich nach außen erstreckende Rastung zum Begrenzen einer axialen Bewegung des Verbindungselements auf der Hülse aufweist.

5. Spritzenanordnung nach Anspruch 1, wobei der Kopplungsmechanismus des Verbindungselements eine Vielzahl von Greifelementen (170) umfasst, die sich vom distalen Ende des Verbindungselements axial erstrecken.

6. Spritzenanordnung nach Anspruch 5, wobei die Greifelemente um das Verbindungselement herum beabstandet sind und wobei jedes der Greifelemente eine sich nach innen erstreckende Rastung zum Koppeln mit dem Behälter umfasst.

7. Spritzenanordnung nach Anspruch 6, wobei die Greifelemente flexibel sind und sich radial nach außen biegen, um mit dem Behälter zu koppeln.

8. Spritzenanordnung nach Anspruch 4, wobei die Hülse eine Innenfläche mit einer ersten Rastung (220) und einer zweiten Rastung (222) zum Zurückhalten der Hülse an dem distalen Ende des Spritzenzylinders aufweist und wobei das Verbindungselement verschiebbar an der Hülse angebracht ist.

9. Verfahren zum Aspirieren und Füllen einer Spritze, umfassend:
Bereitstellen einer Spritzenanordnung gemäß Anspruch 1;
Verbinden des Behälters mit dem Verbindungselement (102, 156) und Durchstechen des Septums (90) an dem Behälter mit der Nadel und Füllen des Spritzenzylinders (12);
Trennen des Behälters von dem Verbindungselement (102, 156) und Zurückziehen des Verbindungselements (102, 156), um die Nadel freizulegen.

10. Verfahren nach Anspruch 9, wobei das Verbindungselement (102, 156) an dem Spritzenzylinder (12) angebracht ist, wobei das Verfahren umfasst:
Verschieben des Verbindungselements (102, 156) aus einer eingezogenen Position, in der die Nadel freiliegt, in eine ausgefahrene Position, in der die Nadel abgedeckt ist;
Koppeln des Verbindungselements (102, 156) mit dem Behälter und Verschieben des Spritzenzylinders (12) relativ zum Verbindungselement (102, 156), wodurch die Nadel das Septum (90) an dem Behälter durchsticht.

## Revendications

1. Ensemble seringue comprenant :
un cylindre de seringue (12) présentant une extrémité proximale (14) et une extrémité distale (16), une embase raccordée à ladite extrémité distale dudit cylindre de seringue ; et
un manchon (104, 200) fixé au cylindre de seringue, ledit manchon présentant une extrémité distale alignée avec l'extrémité distale du cylindre de seringue ;
**caractérisé en ce que** l'ensemble seringue comprend en outre :
un organe de liaison (102, 156), ledit organe de liaison présentant une forme cylindrique et étant monté de manière coulissante sur ledit manchon pour coulisser entre une première position où ladite aiguille est exposée et une deuxième position recouvrant ladite aiguille, ledit organe de liaison présentant une extrémité proximale présentant des organes de retenue internes pour retenir ledit organe de liaison sur ledit manchon et ledit cylindre de seringue, et une extrémité distale présentant un mécanisme (74, 126) de couplage avec un récipient, dans lequel ledit organe de retenue sur ladite extrémité distale dudit organe de liaison comporte un cran (74, 146) s'étendant vers l'intérieur conçu pour engager le récipient et soutenir le récipient lorsque ledit cylindre de seringue et l'aiguille se déplacent par rapport audit organe de liaison pour percer un septum (90) sur ledit récipient.

2. Ensemble seringue selon la revendication 1, dans lequel ledit organe de liaison a une forme cylindrique et est mobile par rapport audit cylindre de seringue.

3. Ensemble seringue selon la revendication 2, dans lequel ledit organe de liaison comporte un collier (58) au niveau de ladite extrémité distale, ledit collier présentant un côté ouvert avec une dimension pour recevoir le récipient pour coulisser latéralement dans ledit organe de liaison, et où ledit cran est prévu sur une surface interne dudit collier.

4. Ensemble seringue selon la revendication 3, dans lequel ledit manchon est positionné sur ladite extrémité distale dudit cylindre de seringue, et où ledit manchon présente un cran s'étendant vers l'extérieur sur une extrémité proximale dudit manchon et un cran s'étendant vers l'extérieur d'une extrémité distale dudit manchon pour limiter le mouvement axial de l'organe de liaison sur ledit manchon.

5. Ensemble seringue selon la revendication 1, dans lequel ledit mécanisme de couplage dudit organe de liaison comporte une pluralité d'organes de préhension (170) s'étendant axialement à partir de ladite extrémité distale dudit organe de liaison.

6. Ensemble seringue selon la revendication 5, dans lequel lesdits organes de préhension sont espacés autour dudit organe de liaison et où chacun desdits organes de préhension comporte un cran s'étendant vers l'intérieur pour le couplage au récipient.

7. Ensemble seringue selon la revendication 6, dans lequel lesdits organes de préhension sont flexibles et se courbent radialement vers l'extérieur pour se coupler avec le récipient.

8. Ensemble seringue selon la revendication 4, dans lequel ledit manchon présente une surface interne avec un premier cran (220) et un deuxième cran (222) pour retenir ledit manchon sur ladite extrémité distale dudit cylindre de seringue, et où ledit organe de liaison est monté de manière coulissante sur ledit manchon.

9. Procédé pour aspirer et remplir une seringue, comprenant
la fourniture d'un ensemble seringue selon la revendication 1 ;
la connexion du récipient audit organe de liaison (102, 156) et le perçage du septum (90) sur ledit récipient avec ladite aiguille et le remplissage dudit cylindre de seringue (12) ;
la séparation dudit récipient dudit organe de liaison (102, 156) et la rétraction dudit organe de liaison (102, 156) pour exposer ladite aiguille.

10. Procédé selon la revendication 9, dans lequel ledit organe de liaison (102, 156) est monté sur ledit cylindre de seringue (12), ledit procédé comprenant :
le coulissement de l'organe de liaison (102, 156) à partir d'une position rétractée où ladite aiguille est exposée à une position étendue recouvrant ladite aiguille ;
le couplage dudit organe de liaison (102, 156) au récipient et le coulissement dudit cylindre de seringue (12) par rapport audit organe de liaison (102, 156) par lequel ladite aiguille perce le septum (90) sur le récipient.
